# EUROPÄISCHE PATENTANMELDUNG

(11) **EP 4 135 346 A2**
(43) Veröffentlichungstag der Anmeldung: **15.02.2023**
(21) Anmeldenummer: 22188157.6
(22) Anmeldetag: 01.08.2022
(51) Int. Cl.: H04R 25/00, A61B 5/00, A61B 5/024

(54) **VERFAHREN ZUR ÜBERPRÜFUNG EINES PPG-SENSORS EINER HÖRVORRICHTUNG UND HÖRVORRICHTUNGSSYSTEM**

(30) Priorität: 13.08.2021 DE 102021208928
(71) Anmelder: Sivantos Pte. Ltd., Singapore 539775 (SG)
(72) Erfinder: GÖKAY, Umut, 55116 Mainz (DE)
(74) Vertreter: FDST Patentanwälte

(57) **Zusammenfassung**

Die Erfindung betrifft ein Verfahren zur Überprüfung eines PPG-Sensors (12) einer Hörvorrichtung (1), die eine wiederaufladbare Batterie (10) aufweist, wobei verfahrensgemäß ein Ladegerät (22) mit einem Aufnahmeraum (26) für die Hörvorrichtung (1) bereitgestellt wird, wobei das Ladegerät (22) eine Testumgebung für den PPG-Sensor (12) aufweist, die Hörvorrichtung (1) in dem Aufnahmeraum (26) positioniert wird, und unter Nutzung der Testumgebung ein Funktionstest des PPG-Sensors (12) durchgeführt wird.

## Beschreibung

Die Erfindung betrifft ein Verfahren zur Überprüfung eines PPG-Sensors einer Hörvorrichtung. Des Weiteren betrifft die Erfindung ein Hörvorrichtungssystem.

Hörvorrichtungen dienen üblicherweise zur Ausgabe eines Tonsignals an das Gehör des Trägers dieser Hörvorrichtung. Die Ausgabe erfolgt dabei mittels eines Ausgabewandlers, meist auf akustischem Weg über Luftschall mittels eines Lautsprechers (auch als "Hörer" oder "Receiver" bezeichnet). Häufig kommen derartige Hörvorrichtungen dabei als sogenannte Hörhilfegeräte (auch kurz: Hörgeräte) zum Einsatz. Dazu umfassen die Hörvorrichtungen normalerweise einen akustischen Eingangswandler (insbesondere ein Mikrofon) und einen Signalprozessor, der dazu eingerichtet ist, das von dem Eingangswandler aus dem Umgebungsschall erzeugte Eingangssignal (auch: Mikrofonsignal) unter Anwendung mindestens eines üblicherweise nutzerspezifisch hinterlegten Signalverarbeitungsalgorithmus derart zu verarbeiten, dass eine Hörminderung des Trägers der Hörvorrichtung zumindest teilweise kompensiert wird. Insbesondere im Fall eines Hörhilfegeräts kann es sich bei dem Ausgabewandler neben einem Lautsprecher auch alternativ um einen sogenannten Knochenleitungshörer oder ein Cochlea-Implantat handeln, die zur mechanischen oder elektrischen Einkopplung des Tonsignals in das Gehör des Trägers eingerichtet sind. Unter dem Begriff Hörvorrichtungen fallen zusätzlich insbesondere auch Geräte wie z.B. sogenannte Tinnitus-Masker, Headsets, Kopfhörer und dergleichen.

Vergleichbar zur fortschreitenden Ausbreitung der Nutzung von Funktionen von sogenannten "wearables", bspw. in Form von Fitnessarmbändern, "smartwatches" und dergleichen, die unter anderem mittels Sensoren Körperfunktionen (bspw. Puls, Bewegung und dergleichen) erfassen, hält auch im Bereich der Hörvorrichtungen die Nutzung von derartigen Funktionen Einzug. Bspw. sollen die Körpertemperatur, der Puls oder dergleichen erfasst werden. Eine solche Zusatznutzung von Hörvorrichtungen bietet sich auch an, da, insbesondere im Fall von Hörhilfegeräten, Hörvorrichtungen auch meist körpernah und häufig auch über einen vergleichsweise langen Zeitraum oder sogar ständig getragen werden.

Zur Messung von Puls kommen - neben der Nutzung der bekannten Brustgurte, die elektrische Signale der Herzmuskelkontraktionen erfassen - auch sogenannte Photoplethysmographie-Sensoren ("PPG-Senoren") zum Einsatz. Das Messprinzip eines solchen PPG-Sensors darauf beruht, dass Körpergewebe mit Licht aus einem oder mehreren vorgegebenen Wellenlängenbereichen (bspw. unterschiedlichen Bändern des Nahinfrarot-Bereichs und/oder des sichtbaren Wellenlängenbereichs) bestrahlt wird und reflektierte oder transmittierte Strahlung erfasst und zur Bestimmung von Gewebeeigenschaften, insbesondere der aktuellen Durchblutung, herangezogen wird. Dabei beeinflusst die erfasste Lichtmenge offensichtlich die Auswertung und Bestimmung der Gewebeeigenschaften. Da Hörvorrichtungen, insbesondere Hörhilfegeräte meist am oder sogar im Ohr getragen werden, sind diese auch erkanntermaßen Körperflüssigkeiten, bspw. Schweiß und/oder Cerumen ausgesetzt, die zu einer Verschlechterung der Funktion des PPG-Sensors führen können.

Der Erfindung liegt die Aufgabe zugrunde, die Funktion einer Hörvorrichtung besser überwachen zu können.

Diese Aufgabe wird erfindungsgemäß gelöst durch ein Verfahren mit den Merkmalen des Anspruchs 1. Außerdem wird diese Aufgabe erfindungsgemäß gelöst durch ein Hörvorrichtungssystem mit den Merkmalen des Anspruchs 11. Vorteilhafte und teils für sich erfinderische Ausführungsformen und Weiterentwicklungen der Erfindung sind in den Unteransprüchen und der nachfolgenden Beschreibung dargelegt.

Das erfindungsgemäße Verfahren dient zur Überprüfung, insbesondere Funktionsüberprüfung eines PPG-Sensors einer Hörvorrichtung, die eine wiederaufladbare Batterie aufweist. Verfahrensgemäß wird hierbei ein Ladegerät mit einem Aufnahmeraum für die Hörvorrichtung bereitgestellt. Vorzugsweise ist das Ladegerät als verschließbare Box ausgeführt, so dass im bestimmungsgemäßen Ladebetrieb der Aufnahmeraum mit der darin angeordneten Hörvorrichtung verschlossen ist. Das Ladegerät weist außerdem eine Testumgebung für den PPG-Sensor auf. Die Hörvorrichtung wird in diesem Aufnahmeraum positioniert und anschließend wird unter Nutzung der Testumgebung ein Funktionstest des PPG-Sensors durchgeführt.

Das Ladegerät mit der Testumgebung schafft somit vorteilhafterweise zumindest näherungsweise wiederholbare - zumindest für den Zweck des hier und im Folgenden beschriebenen Verfahrens hinreichend stabile - und somit vergleichbare Umgebungsbedingungen für die Hörvorrichtung. Dies erleichtert oder ermöglicht sogar zunächst die Funktionsüberprüfung.

In einer bevorzugten Verfahrensvariante wird einem Nutzer der Hörvorrichtung zumindest dann eine Meldung bezüglich der Funktionstüchtigkeit des PPG-Sensors ausgegeben, wenn ein Ergebnis des Funktionstests einen vorgegebenen Grenzwert unterschreitet. In diesem Fall wird dem Nutzer insbesondere eine Warnung ausgegeben, dass der PPG-Sensor nicht (mehr) ordnungsgemäß funktioniert. Da die Funktionstüchtigkeit des PPG-Sensors bei einer Hörvorrichtung häufig aufgrund von Verschmutzung des PPG-Sensors mit Körperflüssigkeiten auftritt, insbesondere aufgrund einer zumindest teilweisen Blockade eines Strahlengangs zwischen einer Lichtquelle und einem Photodetektor des PPG-Sensors, wird optional zusätzlich zu der Warnung auch eine Empfehlung zur Reinigung des PPG-Sensors ausgegeben.

In einer optionalen Verfahrensvariante wird im Rahmen des Funktionstests eine Wandung des Aufnahmeraums als Reflexionsfläche für den PPG-Sensor herangezogen. Insbesondere führt in diesem Fall die Hörvorrichtung den Funktionstest als Selbsttest durch. Vorzugsweise weist die Hörvorrichtung hierzu einen Controller auf, der (insbesondere programm- oder schaltungstechnisch) dazu eingerichtet ist, diesen Selbsttest selbsttätig durchzuführen. Insbesondere sendet der PPG-Sensor mittels seiner Lichtquelle ein Testsignal aus, erfasst mittels seines Photodetektors ein (insbesondere aufgrund einer Reflexion des Testsignals an der Wandung auftretendes) Relexionssignal und bewertet daraufhin die Funktionstüchtigkeit des PPG-Sensors. Die Wandung des Aufnahmeraums weist dabei vorzugsweise vorgegebene Reflexionseigenschaften auf. Beispielsweise weist die Wandung eine spiegelnde Oberfläche auf, um eine möglichst geringe optische Dämpfung (und/oder Streuung) des Testsignals zu bewirken. Gleichermaßen ist aber auch eine weiße (optional glänzende) und/oder matte Oberfläche denkbar. Optional ist dabei denkbar, dass die Reflexionseigenschaften beim Einsetzen der Hörvorrichtung in den Aufnahmeraum seitens des Ladegeräts (bspw. im Rahmen einer Art Pairing) an die Hörvorrichtung übermittelt wird.

Zur Auswertung wird in der vorstehend beschriebene Verfahrensvariante die im Rahmen des Testsignals ausgesendete Lichtmenge mit der für das Reflexionssignal ermittelten Lichtmenge sowie insbesondere einem Grenzwert verglichen. Liegen die ausgesendete und die erfasste Lichtmenge zu weit auseinander (unterschreitet also bspw. eine Differenz dieser beiden Lichtmengen den vorgenannten Grenzwert), wird dies als eingeschränkte Funktion gewertet und vorzugsweise die vorgenannte Warnung ausgegeben.

In einer zweckmäßigen Ausführung weist die Testumgebung für den PPG-Sensor eine Test-Lichtquelle auf. Im Rahmen des Funktionstests wird in einer besonders zweckmäßigen Verfahrensvariante der PPG-Sensor mittels der Test-Lichtquelle beleuchtet und ein daraufhin von dem PPG-Sensor ausgegebenes Maß für eine erfasste Lichtmenge mit einem korrespondierenden Maß der von der Test-Lichtquelle abgestrahlten Lichtmenge verglichen. Dadurch kann die Funktion des PPG-Sensors, insbesondere dessen Photodetektors, unabhängig von der Lichtquelle des PPG-Sensors überprüft werden. Denn es kann davon ausgegangen werden, dass mit hinreichend hoher Wahrscheinlichkeit die Test-Lichtquelle nicht oder gegenüber dem PPG-Sensor deutlich langsamer degradiert (insbesondere kaum Verschmutzung ausgesetzt ist), so dass eine unerwartet geringe erfasste Lichtmenge auf eine Funktionseinschränkung des PPG-Sensors hindeutet.

Bevorzugt wird als Test-Lichtquelle eine zur Lichtquelle des PPG-Sensors gleichartige Test-Lichtquelle, die vorzugsweise das gleiche oder ein zumindest hinreichend ähnliches Lichtspektrum ausstrahlt, herangezogen.

In einer besonders zweckmäßigen Weiterbildung wird die Test-Lichtquelle mit einem vorgegebenen Wert einer elektrischen Steuergröße, insbesondere einem Strom, zur Beleuchtung des PPG-Sensors angesteuert. Diese Steuergröße wird dabei insbesondere als Maß für die abgestrahlte Lichtmenge herangezogen. Als Maß für die erfasste Lichtmenge wird in diesem Fall vorzugsweise ein Photostrom des Photodetektors, der bspw. durch eine oder mehrere Photodioden gebildet ist, herangezogen. Der "Photostrom" ist dabei insbesondere der Strom, der von dem Photodetektor in Reaktion auf einfallendes Licht ausgegeben wird. Optional werden die Werte (oder wenigstens einer) dieser beiden Maße bzw. Größen zur besseren Vergleichbarkeit normiert.

In einer weiteren zweckmäßigen Ausführung umfasst die Testumgebung für den PPG-Sensor einen Test-Photodetektor. Im Rahmen des Funktionstests beleuchtet zweckmäßigerweise der PPG-Sensor, insbesondere mittels seiner Lichtquelle, diesen Test-Photodetektor. Ein daraufhin von dem Test-Photodetektor ausgegebenes Maß für eine erfasste Lichtmenge wird mit einem korrespondierenden Maß der von dem PPG-Sensor abgestrahlten Lichtmenge (bspw. der Steuergröße für die Lichtquelle des PPG-Sensors) verglichen.

Vorzugsweise wird als Test-Photodetektor ein zu dem Photodetektor des PPG-Sensors gleichartiger Photodetektor herangezogen, konkret eingesetzt.

In einer zweckmäßigen Weiterbildung wird der PPG-Sensor, insbesondere dessen Lichtquelle, mit einem vorgegebenen Wert einer elektrischen Steuergröße, insbesondere einem Strom, zur Beleuchtung des Test-Photodetektors angesteuert.

Diese Steuergröße wird dabei als Maß für die abgestrahlte Lichtmenge herangezogen. Entsprechend wird in diesem Fall insbesondere der vorstehend beschriebene Photostrom (hier als "Test-Photostrom") als Maß für die mittels des Test-Photodetektors erfasste Lichtmenge herangezogen. Optional werden die Werte (oder wenigstens einer) dieser beiden Maße bzw. Größen zur besseren Vergleichbarkeit normiert.

In einer besonders zweckmäßigen Weiterbildung weist die Testumgebung sowohl die Test-Lichtquelle als auch den Test-Photodetektor auf. In diesem Fall beleuchten die Lichtquelle des PPG-Sensors den Test-Photodetektor und die Test-Lichtquelle den Phototetektor des PPG-Sensors. Dadurch kann vorteilhafterweise jeweils die Funktion des Photodetektors und die der Lichtquelle unabhängig voneinander überprüft werden - zumindest unter der Annahme, dass sowohl die Test-Lichtquelle als auch der Test-Photodetektor funktionstüchtig sind.

Optional ist der Aufnahmeraum in vorstehendem Fall strahlungsabsorbierend, bspw. schwarz-matt, ausgeführt, um eine Reflexion des von der Lichtquelle des PPG-Sensors ausgestrahlten Lichts auf den "eigenen" Photodetektor zu vermeiden. Alternativ oder zusätzlich erfolgt die jeweilige Beleuchtung zeitlich versetzt, um eine Beeinflussung der jeweils anderen Photodetektors zu vermeiden.

In einer weiteren zweckmäßigen Verfahrensvariante wird im Rahmen eines Vortests für den Funktionstest mittels des PPG-Sensors und/oder des Test-Photodetektors eine Helligkeitsmessgröße als Maß für einen Abstand zwischen dem PPG-Sensor und dem Test-Photodetektor und/oder der Test-Lichtquelle ermittelt. Vorzugsweise wird in diesem Fall der Funktionstest nur dann durchgeführt, wenn die Helligkeitsmessgröße einem vorgegebenen Kriterium genügt. Für den Fall, dass die Testumgebung nur die Test-Lichtquelle umfasst, wird die Helligkeitsmessgröße mittels des PPG-Sensors, insbesondere also dessen Photodetektors, ermittelt. Für den Fall, dass die Testumgebung nur den Test-Photodetektor umfasst, wird mittels des PPG-Sensors, insbesondere mittels dessen Lichtquelle, der Test-Photodetektor beleuchtet. Für den Fall, dass die Testumgebung sowohl die Test-Lichtquelle also auch den Test-Photodetektor umfasst, werden optional zwei Helligkeitsmessgrößen ermittelt und miteinander verglichen, optional gemittelt.

Die vorstehend beschriebene Verfahrensvariante ist insbesondere für den Fall zweckmäßig, dass als Hörvorrichtung Hörhilfegeräte zum Einsatz kommen, optional Hörhilfegeräte mit im Gehörgang zu tragenden Lautsprecher-Einheiten, die den PPG-Sensor tragen. In diesem Fall ist häufig diese Lautsprecher-Einheit nicht in gleichem Maße wie ein vergleichsweise größerer Hörvorrichtungskörper im Aufnahmeraum wiederholbar anordenbar. Die Helligkeitsmessgröße, insbesondere der daraus ableitbare Hinweis auf den Abstand zwischen dem PPG-Sensor und der entsprechenden Test-Einheit, ermöglicht somit eine Überprüfung, ob die Anordnung der Hörvorrichtung, optional der Lautsprecher-Einheit, im aktuellen Fall für die Durchführung des Funktionstests ausreichend ist. Sollte die Helligkeitsmessgröße dem Kriterium nicht genügen, insbesondere deren Wert einen das Kriterium bildenden Grenzwert unterschreiten, wird bspw. ein Hinweis an den Nutzer ausgegeben, dass die Hörvorrichtung erneut oder anders in den Aufnahmeraum eingesetzt werden sollte.

In einer bevorzugten Verfahrensvariante werden die vorstehend beschriebenen Maße und Messgrößen, konkret also das Maß für die erfasste Lichtmenge, das Maß für die abgestrahlte Lichtmenge und/oder die Helligkeitsmessgröße zum Vergleich mit dem jeweils korrespondieren Maß bzw. mit dem vorgegebenen Kriterium, von der Hörvorrichtung an das Ladegerät überm ittelt. Seitens des Ladegeräts werden diese Maße und Messgrößen vorzugsweise mittels eines Controllers, der wiederum dazu eingerichtet ist, die vorstehend beschriebenen Vergleiche und Auswertungen durchzuführen, insbesondere auch die Test-Mittel anzusteuern und/oder auszulesen, mit dem jeweils korrespondierenden Maß bzw. Kriterium verglichen. Anders ausgedrückt wird die Auswertung des Funktionstests auf Seiten des Ladegeräts mittels eines entsprechenden Controllers durchgeführt.

Alternativ kann die Auswertung auch auf Seiten der Hörvorrichtung, vorzugsweise mittels eines entsprechend eingerichteten und ausgebildeten Controllers, durchgeführt werden.

Das erfindungsgemäße Hörvorrichtungssystem weist eine Hörvorrichtung auf, die einen PPG-Sensor und eine wiederaufladbare Batterie umfasst, sowie ein Ladegerät, das einen Aufnahmeraum für die Hörvorrichtung und eine Testumgebung für den PPG-Sensor umfasst. Des Weiteren weist das Hörvorrichtungssystem wenigstens einen Controller auf, der dazu eingerichtet ist, das vorstehend beschriebene Verfahren vorzugsweise selbsttätig, optional in Interaktion mit dem Nutzer, durchzuführen. Bevorzugt handelt es sich bei der Hörvorrichtung und dem Ladegerät um die bzw. das jeweils vorstehend im Rahmen des Verfahrens beschriebenen Geräte. Der Controller ist dabei Teil der Hörvorrichtung oder des Ladegeräts. Vorzugsweise weisen beide jeweils einen Controller auf, die optional dazu eingerichtet sind, zumindest jeweils einen Teil des Funktionstests zu übernehmen. Bspw. ist der Controller der Hörvorrichtung dazu eingerichtet, die Signale des PPG-Sensors umzuformen, bspw. aufzubereiten, und zur Übermittlung an das Ladegerät auszugeben.

Das Hörvorrichtungssystem weist mithin alle sich aus der vorstehenden Beschreibung ergebenden vorteilhaften Weiterbildungen inklusive der sich daraus ergebenden körperlichen Merkmale - bspw. die Test-Lichtquelle und/oder den Test-Photodetektor - gleichermaßen in entsprechenden optionalen Weiterbildungen auf. Die sich daraus ergebenden Vorteile kommen somit auch dem Hörvorrichtungssystem zu.

Die Konjunktion "und/oder" ist hier und im Folgenden insbesondere derart zu verstehen, dass die mittels dieser Konjunktion verknüpften Merkmale sowohl gemeinsam als auch als Alternativen zueinander ausgebildet sein können.

Eine zusätzliche Variante oder auch eine gegenüber dem vorstehend beschriebenen Verfahren eigenständige Erfindung stellt ferner das nachfolgend beschriebene Verfahren dar.
- A: Verfahren zur Überprüfung eines Temperatursensors einer Hörvorrichtung die eine wiederaufladbare Batterie aufweist, wobei verfahrensgemäß
- ein Ladegerät mit einem Aufnahmeraum für die Hörvorrichtung bereitgestellt wird, wobei das Ladegerät einen Test-Temperatursensor aufweist,
- die Hörvorrichtung in dem Aufnahmeraum positioniert wird,
- mittels des Temperatursensors (der Hörvorrichtung) und des Test-Temperatursensors jeweils eine Temperaturmessgröße erfasst wird,
- die beiden Temperaturmessgrößen hinsichtlich ihrer Werte miteinander verglichen werden, und
- in Abhängigkeit von einem Unterschied zwischen den Werten der beiden Temperaturmessgrößen eine weitere Maßnahme ergriffen wird.
- B: Verfahren gemäß Punkt A,
wobei die beiden Temperaturmessgrößen (insbesondere deren Werte) zwischen der Hörvorrichtung und dem Ladegerät übermittelt werden. Vorzugsweise wird die Temperaturmessgröße der Hörvorrichtung an das Ladegerät übermittelt.
- C: Verfahren gemäß Punkt A oder B,
wobei als weitere Maßnahme der Temperatursensor der Hörvorrichtung (insbesondere landläufig ausgedrückt) auf den Wert des Test-Temperatursensors kalibriert (oder auch referenziert) wird. Anders ausgedrückt wird insbesondere also der Temperatursensor der Hörvorrichtung auf den Wert des Test-Temperatursensors gesetzt. Dies erfolgt unter der Annahme, dass der Test-Temperatursensor geringeren Beanspruchungen, die die Messung negativ beeinflussen könnten, ausgesetzt ist und damit im Vergleich zum Temperatursensor "verlässlichere" Werte liefern dürfte.
- D: Verfahren gemäß einem der Punkte A bis C,
wobei anhand einer zeitlichen Entwicklung wenigstens eines der Werte der Temperaturmessgrößen eine Temperaturstabilisierung in dem Aufnahmeraum abgewartet wird. Insbesondere wird also abgewartet, bis sich die Werte des entsprechenden (Test-) Temperatursensors nicht mehr (oder nur in einem vernachlässigbaren Maß) ändern, somit also ein eingeschwungener (d. h. stabiler, vorzugsweise statischer) thermischer Zustand im Aufnahmeraum vorliegt.
- E: Verfahren gemäß einem der Punkte A bis D,
wobei als Temperatursensor der Hörvorrichtung ein resistiver Temperatursensor oder ein Infrarot-Temperatursensor und als Test-Temperatursensor entsprechend umgekehrt ein Infrarot-Temperatursensor bzw. ein resistiver Temperatursensor herangezogen wird. Insbesondere wird als Infrarot-Temperatursensor der Photodetektor des vorstehend beschriebenen PPGSensors herangezogen, der vorteilhafterweise empfindlich für Infrarot-Strahlung ist.
- F: Verfahren gemäß Punkt E,
wobei der Infrarot-Temperatursensor derart angeordnet wird, dass die entsprechende Temperaturmessgröße an der Messstelle des resistiven Temperatursensors erfasst wird. Insbesondere ist der Aufnahmeraum derart gestaltet, dass bei bestimmungsgemäßer Positionierung der Hörvorrichtung in dem Aufnahmeraum der Temperatursensor und der Test-Temperatursensor in Gegenüberstellung angeordnet sind. Optional ist der Aufnahmeraum dazu derart ausgebildet, dass die Hörvorrichtung nur in einer räumlichen Ausrichtung in dem Aufnahmeraum positioniert werden kann. Durch diese Anordnung des Infrarot-Temperatursensors kann dieser - insbesondere da dieser über eine Freistrecke von bis zu einigen (etwa 2 bis 5) Millimetern hinweg die Temperatur erfassen kann - vorteilhafterweise die Temperatur an der gleichen Stelle wie der resistive Temperatursensor erfassen.
- G: Verfahren gemäß einem der Punkte A bis F, insbesondere E oder F,
wobei als weitere Maßnahme ein Antwortverhalten des Temperatursensors auf eine Temperaturveränderung in Abhängigkeit von der zeitlichen Entwicklung des Werts seiner Temperaturmessgröße sowie des Werts des, insbesondere mittels des als Infrarot-Temperatursensor ausgebildeten, Test-Temperatursensors modelliert wird.

Nachfolgend werden Ausführungsbeispiele der Erfindung anhand einer Zeichnung näher erläutert. Darin zeigen:
- Fig. 1: in einer schematischen Seitenansicht eine Hörvorrichtung,
- Fig. 2: in einer schematischen Seitenansicht ein Hörvorrichtungssystem, das die Hörvorrichtung nach Fig. 1 und ein Ladegerät umfasst, und
- Fig. 3-5: in Ansicht gemäß Fig. 2 jeweils ein weiteres Ausführungsbeispiel des Hörvorrichtungssystems.

Einander entsprechende Teile und Größen sind in allen Figuren stets mit gleichen Bezugszeichen versehen.

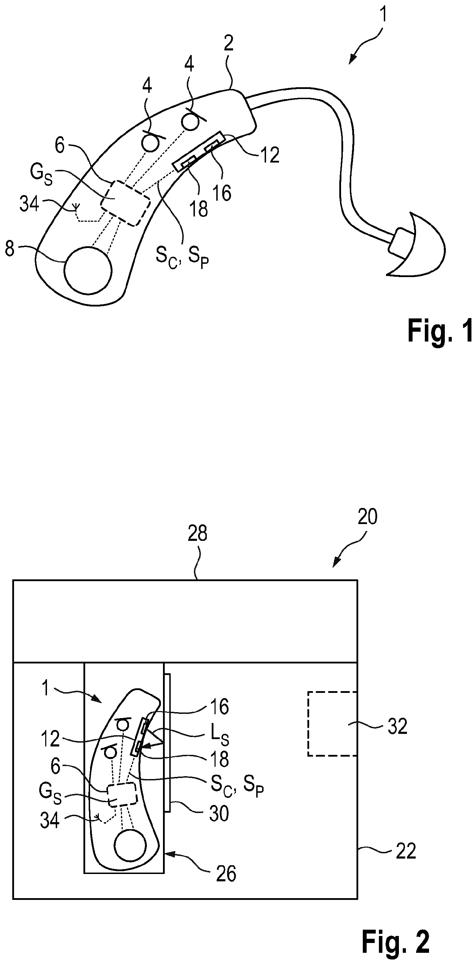

In Fig. 1 ist eine Hörvorrichtung in Form eines Hörhilfegeräts, konkret eines hinter dem Ohr eines Nutzers zu tragenden Hörhilfegeräts (kurz auch als Hörgerät, hier als "BTE 1" bezeichnet), dargestellt. Das BTE 1 umfasst ein Gehäuse 2, in dem elektronische Komponenten des BTE 1 angeordnet sind. Bei diesen elektronischen Komponenten handelt es sich beispielsweise um zwei Mikrofone 4, einen Signalprozessor 6 und ein Batteriemodul 8, das wiederum eine wiederaufladbare Batterie umfasst. Die Mikrofone 4 dienen im bestimmungsgemäßen Betrieb des BTE 1 zum Empfang von Umgebungsschall und Wandlung dessen in elektrische Eingangssignale (auch: "Mikrofonsignale"), die von dem Signalprozessor 6 (auch als "Controller" bezeichnet) verarbeitet (insbesondere gefiltert, frequenzabhängig verstärkt und/oder gedämpft etc.) werden. Die verarbeiteten Eingangssignale werden anschließend als Ausgangssignale an einen nicht dargestellten Lautsprecher ausgegeben und von diesem in Schallsignale gewandelt und an das Gehör des Nutzers weitergegeben.

Das BTE 1 weist außerdem einen Photoplethysmographie-Sensor, kurz "PPG-Sensor 12" auf, der im vorliegenden Ausführungsbeispiel ebenfalls im Gehäuse 2 eingesetzt ist. Der PPG-Sensor 12 dient dazu, bspw. den Puls, optional auch die Sauerstoffsättigung des Nutzers des BTE 1 zu ermitteln. Dazu umfasst der PPG-Sensor 12 eine Lichtquelle, im vorliegenden Fall eine LED-Einheit 16, die dazu eingerichtet ist, Licht mehrerer Frequenzbänder, üblicherweise aber zumindest Licht im Nah-Infrarot-Bereich, auszugeben. Außerdem umfasst der PPG-Sensor 12 einen Lichtsensor 18 ("Photodetektor"), mittels dessen eingestrahltes Licht erfasst wird. Im bestimmungsgemäßen Betrieb erfasst der Lichtsensor 18 Strahlung, die von der LED-Einheit 16 ausgegeben und von Körpergewebe, bspw. einer Gefäßwand eines Blutgefäßes des Nutzers reflektiert wird. Anhand des dabei erfassten Intensitätsverlaufs kann dann bspw. der Puls des Nutzers bestimmt werden.

In Fig. 2 ist ein Hörvorrichtungssystem 20 näher dargestellt. Dieses umfasst das vorstehend beschriebene BTE 1 sowie ein Ladegerät 22. Letzteres dient zum Wiederaufladen der wiederaufladbaren Batterie des BTE 1. Dazu weißt das Ladegerät 22 einen Aufnahmeraum 26 auf, in dem das BTE 1 im bestimmungsgemäßen Ladezustand (s. Fig. 2) positioniert ist. Das Ladegerät 22 stellt dabei eine Testumgebung für das BTE 1, konkret für dessen PPG-Sensor 12 bereit. Außerdem weist das Ladegerät 22 einen Deckel 28 auf, mittels dessen der Aufnahmeraum 26 verschließbar ist.

Ein Beispiel eines Verfahrens für eine Funktionsüberprüfung des PPG-Sensors 12 wird im Folgenden anhand von Fig. 2 näher beschrieben. Dabei wird von dem BTE 1, konkret mittels dessen Signalprozessors 6, ein Selbsttest des PPG-Sensors 12 durchgeführt. Die Testumgebung dient im Beispiel gemäß Fig. 2 dazu, möglichst wiederholbare und stabile Bedingungen für den Selbsttest des PPG-Sensors 12 bereitzustellen. Hierzu ist eine Wandung des Aufnahmeraums 26, die im bestimmungsgemäßen Einlegezustand des BTE 1 im Aufnahmeraum 26 mit einer reflektierenden Oberfläche 30 vorgegebener "Qualität" ausgestattet. Bspw. ist diese Oberfläche 30 weiß, aber nicht glänzend ausgeführt, um eine Lichtstreuung in möglichst alle Richtungen zu ermöglichen ("diffuse Streuung/Reflexion"). Im Rahmen des Selbsttests steuert der Signalprozessor 6 den PPG-Sensor 12 an, um mittels der LED-Einheit 16 ein Lichtsignal ("Testsignal") Ls mit einer vorgegebenen Lichtmenge (oder auch: Strahlungsintensität) auszugeben. Mittels des Lichtsensors 18 wird zumindest ein von der Oberfläche 30 reflektierter Teil des Lichtsignals Ls erfasst. Als Maß für die erfasste Lichtmenge wird ein von dem Lichtsensor 18 daraufhin ausgegebener Photostrom S_{P} herangezogen. Dessen Wert wird mit einem Maß für die abgestrahlte Lichtmenge, hier konkret eine Steuergröße, insbesondere ein Steuerstrom Sc, mittels dessen die LED-Einheit 15 angesteuert wird, verglichen. Überschreitet der Wert des Photostroms S_{P} einen Grenzwert Gs, der abhängig von der Beschaffenheit der Oberfläche 30 vorgegeben ist, kann davon ausgegangen werden, dass der PPG-Sensor 12 funktionstüchtig ist. Wird dieser Grenzwert Gs unterschritten, gibt der Signalprozessor 6 eine Meldung an den Nutzer des BTE 1 aus. In dieser Meldung weist der Signalprozessor 6 den Nutzer daraufhin, dass der PPG-Sensor 12 nicht bestimmungsgemäß funktioniert, bspw. verschmutzt ist.

Optional erfolgt der Vergleich mit dem Grenzwert Gs in einem Controller 32 des Ladegeräts 22. In diesem Fall überträgt der Signalprozessor den Wert des Steuerstroms Sc und des Photostroms S_{P} mittels eines Transmitters 34 an den Controller 32 des Ladegeräts 22.

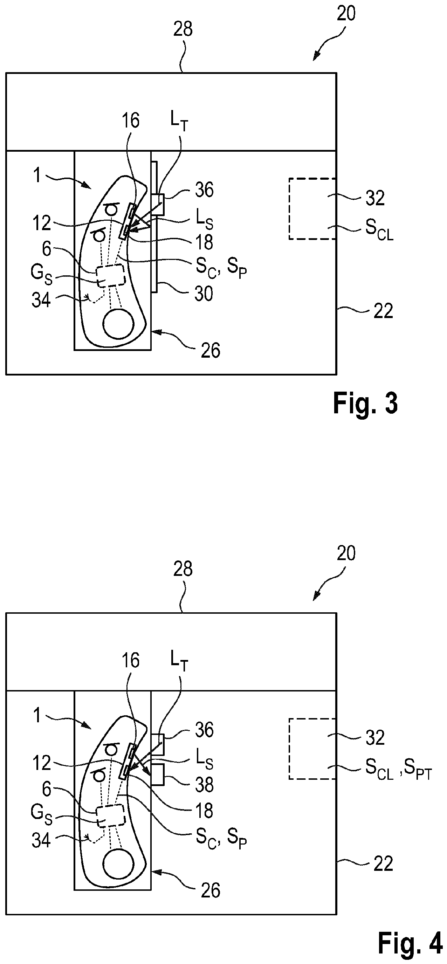

In Fig. 3 ist ein alternatives Ausführungsbeispiel des Hörvorrichtungssystems 20 sowie des mittels dieses durchgeführten Verfahrens zur Funktionsüberprüfung des PPG-Sensors 12 beschrieben. Neben der vorstehend beschriebenen Oberfläche 30 weist das Ladegerät 22 auch eine Test-Lichtquelle 36. Diese ist gleichartig, konkret baugleich, zu der LED-Einheit 16 des PPG-Sensors 12 und kann somit Licht der gleichen Wellenlänge(n) abstrahlen. Außerdem ist die Test-Lichtquelle 36 derart im Aufnahmeraum 26 positioniert, dass diese bei bestimmungsgemäßer Positionierung des BTE 1 in Gegenüberstellung zu dem PPG-Sensor 12 angeordnet ist.

Diese Test-Lichtquelle 36 wird optional zusätzlich oder alternativ zu dem vorstehend beschriebenen Vorgehen dazu genutzt, im Rahmen der Funktionsüberprüfung den Lichtsensor 18 separat zu testen. Dazu strahlt das Ladegerät 22 mittels der Test-Lichtquelle 36 ein Test-Lichtsignal L_{T} in Richtung des PPG-Sensors 12 ab. Wird dieses mittels des Lichtsensors 18 erfasst, sendet das BTE 1 den ermittelten Photostrom S_{P} an den Controller 32 des Ladegeräts 22. Dieser vergleicht den Photostrom S_{P} (dessen Wert) mit dem eigenen, an die Test-Lichtquelle 36 ausgegebenen, Steuerstrom S_{CL} (konkret dessen Wert). Sind die Werte beider Größen hinreichend ähnlich, schließt der Controller 32 auf die Funktionstüchtigkeit des Lichtsensors 18. Ansonsten gibt der Controller 32 eine Warnung aus. Wird bei funktionstüchtigem Lichtsensor 18 nachfolgend der vorstehend beschriebene Selbsttest durchgeführt und dabei der Grenzwert Gs unterschritten, deutet dies auf eine Funktionsstörung der LED-Einheit 16 hin. Eine entsprechende Warnung wird daraufhin ausgegeben.

In Fig. 4 ist ein alternatives Ausführungsbeispiel des Hörvorrichtungssystems 20 sowie des mittels dieses durchgeführten Verfahrens zur Funktionsüberprüfung des PPG-Sensors 12 beschrieben. Neben der vorstehend zu Fig. 3 beschriebenen Test-Lichtquelle 36 weist das Ladegerät 22 auch einen Test-Photodetektor (hier als "Test-Lichtsensor 38" bezeichnet) auf. Dieser ist gleichartig, konkret baugleich, zu dem Lichtsensor18 des PPG-Sensors 12 und weist somit die gleiche Detektionscharakteristik auf. Auch der Test-Lichtsensor 38 ist derart im Aufnahmeraum 26 positioniert, dass dieser bei bestimmungsgemäßer Positionierung des BTE 1 in Gegenüberstellung zu dem PPG-Sensor 12 angeordnet ist.

Neben dem anhand von Fig. 3 beschriebenen Vorgehen wird im Rahmen der Funktionsüberprüfung das Lichtsignal Ls von der LED-Einheit 16 ausgesendet und mittels des Test-Lichtsensors 38 zumindest zu Teilen empfangen. Ein korrespondierender Test-Photostrom S_{PT} wird vom Controller 32 mit dem Steuerstrom Sc für die LED-Einheit 16, der vom BTE 1 an den Controller 32 übermittelt wurde, verglichen. So kann also die Funktion der LED-Einheit 16 sowie des Lichtsensors 18 unabhängig voneinander geprüft werden.

In einer optionalen Variante ist der Controller 32 dazu eingerichtet, einen Selbsttest für die Test-Lichtquelle 36 und den Test-Lichtsensor 38 durchzuführen, indem diese vergleichbar zu dem PPG-Sensor betrieben werden, bspw. wenn das BTE 1 nicht eingelegt ist. In diesem Fall wird vergleichbar zu dem anhand von Fig. 2 beschriebenen Selbsttest vorgegangen.

Optional sind mehrere Test-Lichtquellen 36 im Aufnahmeraum 26 angeordnet. Um zu ermitteln, welcher Test-Lichtquelle 36 der PPG-Sensor 12 am "besten", d. h. konkret am nähesten, gegenüberliegend angeordnet ist, werden die einzelnen Test-Lichtquellen 36 nacheinander angesteuert und die jeweils erfasste Intensität erfasst. Die Test-Lichtquelle 36, die die "stärkste" Reaktion hervorruft, wird als nächstliegendste angenommen.

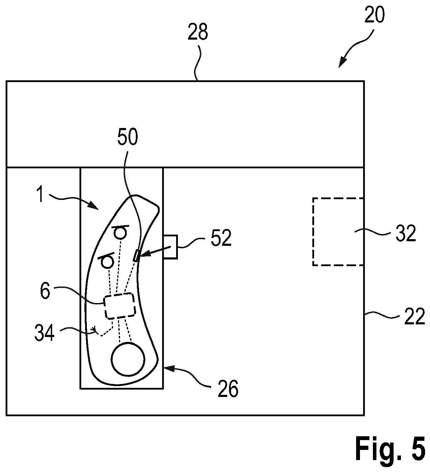

In Fig. 5 ist ein weiteres Ausführungsbeispiel des Hörvorrichtungssystems 20 dargestellt. Das BTE 1 umfasst dabei einen Temperatursensor 50, der als resistiver Temperatursensor ausgebildet ist. Dieser dient, im bestimmungsgemäßen Tragezustand die Körpertemperatur des Nutzers des BTE 1 zu ermitteln. Das Ladegerät 22 umfasst einen Infrarot-Temperatursensor, kurz IR-Sensor 52. Dieser ist wie vorstehend für die Test-Lichtquelle 36 beschrieben, ebenfalls in Gegenüberstellung zu dem Temperatursensor 50 angeordnet. Mittels des IR-Sensors 52 wird nach dem Einlegen des BTE 1 in das Ladegerät 22, konkret in den Aufnahmeraum 26, die Temperatur an der Stelle des Temperatursensors 50 erfasst. Nach einer Angleichzeit, deren Ende daran erkannt wird, dass sich die erfasste Temperaturwerte nicht mehr ändern, werden die mittels des Temperatursensors 50 und des IR-Sensors 52 erfassten Temperaturwerte miteinander verglichen. Weichen diese voneinander ab, wird der Temperatursensor 50 auf den Wert des IR-Sensors 52 gesetzt, mithin auf diesen "kalibriert".

Der Gegenstand der Erfindung ist nicht auf die vorstehend beschriebenen Ausführungsbeispiele beschränkt. Vielmehr können weitere Ausführungsformen der Erfindung von dem Fachmann aus der vorstehenden Beschreibung abgeleitet werden. Insbesondere können die anhand der verschiedenen Ausführungsbeispiele beschriebenen Einzelmerkmale der Erfindung und deren Ausgestaltungsvarianten auch in anderer Weise miteinander kombiniert werden.

### Bezugszeichenliste

- 1: BTE
- 2: Gehäuse
- 4: Mikrofon
- 6: Signalprozessor
- 8: Batteriemodul
- 12: PPG-Sensor
- 16: LED-Einheit
- 18: Lichtsensor
- 20: Hörvorrichtungssystem
- 22: Ladegerät
- 26: Aufnahmeraum
- 28: Deckel
- 30: Oberfläche
- 32: Controller
- 34: Transmitter
- 36: Test-Lichtquelle
- 38: Test-Lichtsensor
- 50: Temperatursensor
- 52: IR-Sensor

- Ls: Lichtsignal
- L_{T}: Test-Lichtsignal
- Sc: Steuerstrom
- S_{P}: Photostrom
- Gs: Grenzwert
- S_{CL}: Steuerstrom
- S_{PT}: Test-Photostrom

## Patentansprüche

1. Verfahren zur Überprüfung eines PPG-Sensors (12) einer Hörvorrichtung (1), die eine wiederaufladbare Batterie (10) aufweist, wobei verfahrensgemäß
- ein Ladegerät (22) mit einem Aufnahmeraum (26) für die Hörvorrichtung (1) bereitgestellt wird, wobei das Ladegerät (22) eine Testumgebung für den PPG-Sensor (12) aufweist,
- die Hörvorrichtung (1) in dem Aufnahmeraum (26) positioniert wird, und
- unter Nutzung der Testumgebung ein Funktionstest des PPG-Sensors (12) durchgeführt wird.

2. Verfahren nach Anspruch 1,
wobei einem Nutzer der Hörvorrichtung (1) zumindest dann eine Meldung bezüglich der Funktionstüchtigkeit des PPG-Sensors (12) ausgegeben wird, wenn ein Ergebnis des Funktionstests einen vorgegebenen Grenzwert (Gs) unterschreitet.

3. Verfahren nach Anspruch 1 oder 2,
wobei im Rahmen des Funktionstests eine Wandung des Aufnahmeraums (26) als Reflexionsfläche für den PPG-Sensor (12) herangezogen wird.

4. Verfahren nach Anspruch 1 oder 2,
wobei die Testumgebung für den PPG-Sensor (12) eine Test-Lichtquelle (36), insbesondere eine zu einer Lichtquelle (16) des PPG-Sensors (12) gleichartige Test-Lichtquelle (36), umfasst, wobei im Rahmen des Funktionstests der PPG-Sensor (12) mittels der Test-Lichtquelle (36) beleuchtet und ein daraufhin von dem PPG-Sensor (12) ausgegebenes Maß (S_{P}) für eine erfasste Lichtmenge mit einem korrespondierenden Maß (S_{CL}) der von der Test-Lichtquelle (36) abgestrahlten Lichtmenge verglichen wird.

5. Verfahren nach Anspruch 4,
wobei die Test-Lichtquelle (36) mit einem vorgegebenen Wert einer elektrischen Steuergröße (S_{CL}), insbesondere einem Strom, zur Beleuchtung des PPG-Sensors (12) angesteuert wird, und wobei die Steuergröße (S_{CL}) als Maß für die abgestrahlte Lichtmenge herangezogen wird.

6. Verfahren nach einem der Ansprüche 1, 2, 4 oder 5,
wobei die Testumgebung für den PPG-Sensor (12) einen Test-Photodetektor (38), insbesondere eine zu einem Photodetektor (18) des PPG-Sensors (12) gleichartigen Test-Photodetektor (38), umfasst, wobei im Rahmen des Funktionstests der PPG-Sensor (12) den Test-Photodetektor (38) beleuchtet und ein daraufhin von dem Test-Photodetektor (38) ausgegebenes Maß (S_{PT}) für eine erfasste Lichtmenge mit einem korrespondierenden Maß (Sc) der von dem PPG-Sensor (12) abgestrahlten Lichtmenge verglichen wird.

7. Verfahren nach Anspruch 6,
wobei der PPG-Sensor (12), insbesondere eine Lichtquelle (16) des PPG-Sensors (12), mit einem vorgegebenen Wert einer elektrischen Steuergröße (Sc), insbesondere einem Strom, zur Beleuchtung des Test-Photodetektors (38) angesteuert wird, und wobei die Steuergröße (Sc) als Maß für die abgestrahlte Lichtmenge herangezogen wird.

8. Verfahren nach Anspruch 4 und 6.

9. Verfahren nach einem der Ansprüche 4 bis 8,
wobei im Rahmen eines Vortests für den Funktionstest mittels des PPG-Sensors (12) und/oder des Test-Photodetektors (38) eine Helligkeitsmessgröße als Maß für einen Abstand zwischen dem PPG-Sensor (12) und dem Test-Photodetektor (38) und/oder der Test-Lichtquelle (36) ermittelt wird, insbesondere wobei der Funktionstest nur dann durchgeführt wird, wenn die Helligkeitsmessgröße einem vorgegebenen Kriterium genügt.

10. Verfahren nach einem der Ansprüche 4 bis 9,
wobei das Maß für die erfasste Lichtmenge (S_{P}), das Maß für die abgestrahlte Lichtmenge (Sc) und/oder die Helligkeitsmessgröße zum Vergleich mit dem jeweils korrespondieren Maß (Sc, S_{P}, S_{CL}, S_{PT}) bzw. mit dem vorgegebenen Kriterium von der Hörvorrichtung (1) an das Ladegerät (22) übermittelt und seitens des Ladegeräts (22) mittels eines hierzu eingerichteten Controllers (32) mit dem jeweils korrespondierenden Maß (Sc, S_{P}, S_{CL}, S_{PT}) bzw. Kriterium verglichen wird.

11. Hörvorrichtungssystem (20) aufweisend eine Hörvorrichtung (1), die einen PPG-Sensor (12) und eine wiederaufladbare Batterie (10) umfasst, sowie ein Ladegerät (22), das einen Aufnahmeraum (26) für die Hörvorrichtung (1) und eine Testumgebung für den PPG-Sensor (12) umfasst, und wenigstens einen Controller (6, 32), der dazu eingerichtet ist das Verfahren nach einem der Ansprüche 1 bis 10 durchzuführen.
